# EUROPÄISCHE PATENTANMELDUNG

(11) **EP 1 442 731 A2**
(43) Veröffentlichungstag der Anmeldung: **04.08.2004**
(21) Anmeldenummer: 04000684.3
(22) Anmeldetag: 15.01.2004
(51) Int. Cl.: A61F 5/01

(54) **Knieorthese zur Stabilisierung des Gelenks**

(30) Priorität: 27.01.2003 DE 20301399 U
(71) Anmelder: Bort GmbH, 71384 Weinstadt-Benzach (DE)
(72) Erfinder: Jaekel, Udo, 89075 Ulm (DE)
(74) Vertreter: Patentanwälte Ruff, Wilhelm, Beier, Dauster & Partner

(57) **Zusammenfassung**

Eine Knieorthese (1) zur Stabilisierung des Kniegelenks mit Rahmenteilen (2,3) aus einem metallischen Werkstoff, die sich in einer Funktionsposition oberhalb und unterhalb des Kniegelenks jeweils längs eines Beinabschnittes erstrecken und mit zumindest jeweils einem Verschlussmittel (6,7,8,9,14) befestigt sind, das den Beinabschnitt formschlüssig lösbar umschlingt, wobei die einem Beinabschnitt zugeordneten Rahmenteile (2,3) durch jeweils zumindest eine quer zum Beinabschnitt verlaufende feste Verbindungsspange (4,5) verbunden sind und zwischen den ober- und unterhalb des Kniegelenks angeordneten Rahmenteilen (2,3) ein- oder beidseitig des Kniegelenks jeweils schwenkbewegliche Verbindungsmittel (12) vorgesehen sind, deren Rotationsachsen im wesentlichen mit einer Hauptbewegungsachse des Kniegelenks übereinstimmen, mit zumindest einer Verbindungsspange (4,5), die als separates Bauteil aus einem Werkstoff vorgesehen ist, der in einem vorübergehend plastifizierten Zustand unmittelbar an einen Beinquerschnitt des jeweiligen Beinabschnittes anpassbar ist.

## Beschreibung

Die Erfindung betrifft eine Knieorthese zur Stabilisierung des Kniegelenks mit Rahmenteilen aus einem metallischen Werkstoff, die sich in einer Funktionsposition oberhalb und unterhalb des Kniegelenks jeweils längs eines Beinabschnittes erstrecken und mit zumindest jeweils einem Verschlussmittel befestigt sind, das den Beinabschnitt formschlüssig lösbar umschlingt, wobei die einem Beinabschnitt zugeordneten Rahmenteile durch jeweils zumindest eine quer zum Beinabschnitt verlaufende feste Verbindungsspange verbunden sind, und zwischen den ober- und unterhalb des Kniegelenks angeordneten Rahmenteilen einoder beidseitig des Kniegelenks jeweils schwenkbewegliche Verbindungsmittel vorgesehen sind, deren Rotationsachsen im wesentlichen mit einer Hauptbewegungsachse des Kniegelenks übereinstimmen.

Derartige Knieorthesen sind aus dem Stand der Technik in vielfältigen Ausführungsformen bekannt. Sie dienen zum Stützen eines Kniegelenks eines Patienten, insbesondere zur Stabilisierung des Kniegelenks nach Verletzungen oder operativen Eingriffen. Um die angestrebte Stabilisierungswirkung zu erreichen, besteht eine Knieorthese aus im wesentlichen steifen und aus metallischem Werkstoff hergestellten Rahmenteilen, die sich in einer Funktionsposition oberhalb und unterhalb des Kniegelenks jeweils längs eines Beinabschnittes erstrecken. Die einem Beinabschnitt zugeordneten Rahmenteile sind jeweils über zumindest eine Verbindungsspange miteinander verbunden. Weiterhin sind Verschlussmittel, insbesondere Textilbänder mit Klettverschlüssen vorgesehen, die an den Rahmenteilen angebracht sind und den jeweiligen Beinabschnitt formschlüssig lösbar umschlingen. Die oberhalb und unterhalb des Kniegelenks angeordneten Rahmenteile sind mit schwenkbeweglichen Verbindungsmitteln, insbesondere Drehgelenken, Kugelgelenken, polizentrischen Gelenken oder Zahnradgelenken miteinander verbunden. Dadurch wird eine Kraftübertragung zwischen den ober- und unterhalb des Kniegelenks gelegenen Beinabschnitten ermöglicht, wodurch ein erheblicher Anteil der über das Kniegelenk zu übertragenden Kräfte zur Entlastung des Kniegelenks über die Knieorthese übertragen werden können. Darüber hinaus wird der naturgemäß verhältnismäßig große Bewegungsbereich des Kniegelenks auf eine Hauptbewegungsrichtung beschränkt.

Eine Anpassung einer aus dem Stand der Technik bekannten Knieorthese wird insbesondere über die Verschlussmittel, die häufig als Klettverschlussbänder ausgeführt sind, erreicht. Weiterhin können auch die aus metallischem Werkstoff hergestellten Rahmenteile und die Verbindungsspangen durch plastische Deformation ungefähr an die Bedürfnisse eines Benutzers angepasst werden. Der dabei zu erreichende Grad der Anpassung ist jedoch deutlich eingeschränkt, da sich bei metallischen Werkstoffen eine Deformation aus einem elastischen Deformationsanteil und einem plastischen Deformationsanteil zusammensetzt, wodurch eine exakte Anpassung aufwendig und mühsam wird. Die aus dem Stand der Technik bekannten Knieorthesen erlauben somit lediglich eine unvollkommene Anpassung an die Bedürfnisse des Benutzers, was insbesondere durch die eingeschränkte Auswahl der in Frage kommenden Werkstoffe bestimmt ist.

Die Aufgabe der Erfindung besteht darin, eine Knieorthese der eingangs genannten Art zu schaffen, bei der eine verbesserte Anpassung an die Bedürfnisse des Benutzers ermöglicht wird.

Diese Aufgabe wird dadurch gelöst, dass zumindest eine Verbindungsspange als separates Bauteil aus einem Werkstoff vorgesehen ist, der in einem vorübergehend plastifizierten Zustand unmittelbar an einen Beinquerschnitt des jeweiligen Beinabschnittes anpassbar ist. Durch eine vorübergehende und reversible Plastifizierung der Verbindungsspange, insbesondere durch Zufuhr von Wärmeenergie, lässt sich eine nahezu kraftfreie Anpassung der Verbindungsspange an den jeweiligen Beinquerschnitt des Benutzers erreichen. Durch den plastischen Zustand sind nur geringe Deformationskräfte notwendig. Geeignete Werkstoffe kehren nach kurzer Zeit aus dem plastifizierten in einen festen und stabilen Ausgangszustand zurück und bieten dann wieder die für eine sinnvolle Nutzung der Knieorthese notwendige Festigkeit. Insbesondere nach operativen Eingriffen am Kniegelenk weist der Benutzer einer Knieorthese üblicherweise eine niedrige Schmerzakzeptanz auf, die eine Anpassung und Benutzung der Knieorthese erschwert. Da gerade zu diesem Zeitpunkt große Kräfte und Momente vom Kniegelenk ferngehalten werden müssen, kann die erfindungsgemäße Verbindungsspange dazu beitragen, dass sich durch die sorgfältige Anpassung der Rahmenteile und der Verbindungsspangen eine flächige Auflage der Knieorthese an den Beinabschnitten und dadurch eine einwandfreie Kraftübertragung ergibt.

In Ausgestaltung der Erfindung dient die anpassbare Verbindungsspange als Tibiaspange. Eine Tibiaspange verläuft an einem Unterschenkelabschnitt zwischen Kniegelenk und Sprunggelenk quer zu einem Schienbeinknochen. Im Gegensatz zu einem zwischen Kniegelenk und Hüftgelenk befindlichem Oberschenkelabschnitt, bei dem der Knochen von einem stark ausgeprägtem Muskelgewebe umschlossen ist, befindet sich beim Unterschenkelabschnitt insbesondere der Schienbeinknochen lediglich unmittelbar unter einer dünnen Hautschicht. Die entsprechende Verbindungsspange, die in Anbetracht der zu übertragenden Kräfte eine gewisse Mindeststeifigkeit aufweisen muss, liegt dadurch nahezu unmittelbar auf dem Schienbeinknochen auf und ruft bei einer ungenügenden Anpassung an die dort vorliegende Beinkontur schmerzhafte Druckstellen hervor.

In weiterer Ausgestaltung der Erfindung ist der Werkstoff ein thermoplastischer Kunststoff mit einer Faserverstärkung, insbesondere mit einem Flächengebilde aus Kohlefaser. Die Verwendung eines thermoplastischen Kunststoffs für die Verbindungsspange eröffnet die Möglichkeit zur Plastifizierung des Werkstoffes durch Wärmezufuhr, die in Anbetracht entsprechend ausgewählter Werkstoffeigenschaften nur eine verhältnismäßig geringe Temperaturdifferenz zur Körpertemperatur erfordert. Die Verwendung einer Faserverstärkung erlaubt eine dünnwandige Gestaltung der Verbindungsspange bei hoher Festigkeit. Dazu wird insbesondere ein Flächengebilde aus Kohlefasern eingesetzt, bei dem die Kohlefasern insbesondere gewebt, gewirkt oder in anderer Weise zu einer flächigen Matrix verbunden werden. Diese Matrix erschwert eine Deformation der Verbindungsspange abseits einer vordefinierten Vorzugsrichtung.

In weiterer Ausgestaltung der Erfindung ist zumindest eine Verbindungsspange lösbar mit den Rahmenteilen verbunden. Dadurch kann eine besonders einfache Handhabung der Verbindungsspange für den Plastifizierungs- und Anpassungsprozess erzielt werden. Weiterhin lassen sich darüber auch unterschiedliche Beinquerschnitte mit entsprechend größenmäßig abgestuften Verbindungsspangen abdecken.

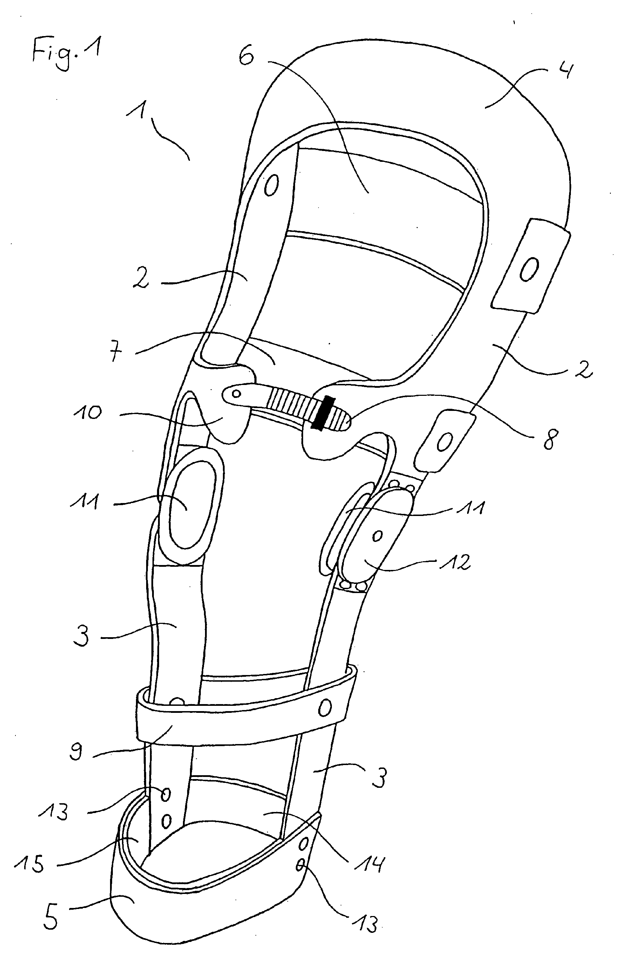

Weitere Vorteile und Merkmale der Erfindung ergeben sich aus den Ansprüchen und der nachfolgenden Beschreibung einer bevorzugten Ausführungsform der Erfindung, die anhand der einzigen Figur dargestellt ist.

Die einzige Figur zeigt eine bevorzugte Ausführungsform der erfindungsgemäßen Knieorthese in perspektivischer Darstellung.

Eine Knieorthese 1 weist zwei Oberschenkelrahmenteile 2 auf, die mit einer Oberschenkelverbindungsspange 4 einstückig verbunden sind und die sich in einer Funktionsposition längs eines nicht dargestellten Oberschenkels eines Benutzers erstrecken. Für eine formschlüssige Befestigung an einem nicht dargestellten Oberschenkelabschnitt ist ein Oberschenkelzugband 6 vorgesehen, das mit einem nicht näher dargestellten Klettverschluss weitenverstellbar auf einen Umfang des Oberschenkelabschnittes angepasst werden kann. Das Oberschenkelzugband 6 ist benachbart zur Oberschenkelverbindungsspange 4 fest an den Oberschenkelrahmenteilen 2 angebracht. Die Oberschenkelrahmenteile 2 sind über nicht näher dargestellte Bewegungsgelenke 12 mit Unterschenkelrahmen 3 verbunden, wobei die Bewegungsgelenke 12 lediglich eine Relativbewegung der Rahmenteile 2, 3 um eine Rotationsachse zulassen, die einer Hauptbewegungsrichtung eines Kniegelenks des Benutzers entspricht. Für eine vorteilhafte Übertragung am Kniegelenk auftretender Kräfte sind an den Oberschenkelrahmenteilen 2 in der Nähe der Bewegungsgelenke 12 hervorspringende Stützzungen 10 vorgesehen, die den Oberschenkel mittels einer Weitenverstellung 8 formschlüssig umschließen. Zusätzlich ist in der Nähe der Stützzungen 10 ein Oberschenkelstützband 7 vorgesehen, das analog dem Oberschenkelzugband 6 als weitenverstellbares Klettband den Oberschenkel formschlüssig umfasst und ebenfalls fest an den Oberschenkelrahmenteilen 2 angebracht ist.

An den Unterschenkelrahmenteilen 3 ist ein Unterschenkelstützband 9 vorgesehen, das als umlaufende Schlaufe mit einem nicht näher dargestellten Klettverschluss einen Unterschenkel des Benutzers weitenverstellbar formschlüssig umfasst. Das Unterschenkelstützband 9 ist an dem Unterschenkelrahmenteil 3 jeweils fest angebracht. An einem dem Bewegungsgelenk 12 abgewandtem Endbereich der Unterschenkelrahmenteile 3 ist eine Unterschenkelverbindungsspange 5 angebracht, die aus einem thermoplastischen Werkstoff mit eingelagerter Kohlefasermatrix hergestellt ist. Diese Unterschenkelverbindungsspange 5 ist über beidseitig vorgesehene Spangenverschlüsse 13 fest mit den Unterschenkelrahmenteilen 3 verbunden. Die Spangenverschlüsse 13 sind beim dargestellten Ausführungsbeispiel durch Nietverbindungen gebildet. Die Unterschenkelverbindungsspange 5 erstreckt sich dabei nahezu halbkreisförmig von einem Unterschenkelrahmenteil 3 zum anderen. Um eine feste Anlage der Unterschenkelverbindungsspange 5 an einem nicht dargestellten Unterschenkelabschnitt des Benutzers zu gewährleisten, ist ein Unterschenkelzugband 14 vorgesehen. Das Unterschenkelzugband 14 erlaubt mittels einer weitenverstellbaren Klettverschlussanordnung eine individuelle Anpassung an einen Unterschenkelquerschnitt des Benutzers. Auf Höhe der Bewegungsgelenke 12 ist an den dem Kniegelenk zugewandten Innenflächen der Oberschenkel- beziehungsweise Unterschenkelrahmenteile 2, 3 beidseitig jeweils ein Kniegelenkpolster 11 vorgesehen, das einen direkten Kontakt des Bewegungsgelenks 12 mit dem Kniegelenk verhindert.

Um eine vorteilhafte Anpassung der Knieorthese 1 an nicht dargestellte Ober- und Unterschenkelabschnitte ober- bzw. unterhalb eines Kniegelenks des Benutzers zu erreichen, werden in einem ersten Schritt alle Zug- und Stützbänder 6, 7, 8, 9, 14 mittels der dafür vorgesehenen Verschlüsse geöffnet. Anschließend wird in einem zweiten Schritt eine Plastifizierung des Werkstoffes der Unterschenkelverbindungsspange 5 durch Zufuhr von Wärme erzielt. In einem dritten Schritt wird nun die Knieorthese 1 auf die zu stabilisierenden Ober- und Unterschenkelabschnitte des Benutzers aufgelegt und das Unterschenkelzugband 14 geschlossen, so dass sich bedingt durch den Unterschenkelquerschnitt eine Deformationskraft auf die Unterschenkelverbindungsspange 5 ergibt. Diese Deformationskraft ermöglicht es dem vorübergehend plastifizierten Material, eine besonders vorteilhafte und gut angepasste Form zu finden. Nach Übergang der Unterschenkelverbindungsspange 5 aus dem plastifizierten in einen mechanisch stabilen Zustand können die weiteren Zug- und Stützbänder 6, 7, 8, 9, 14 geschlossen werden und eine Anpassung der Knieorthese 1 durch die Weitenverstellung 8 vorgenommen werden.

### Bezugszeichenliste:

- 1.: Knieorthese
- 2.: Oberschenkelrahmenteil
- 3.: Unterschenkelrahmenteil
- 4.: Oberschenkelverbindungsspange
- 5.: Unterschenkelverbindungsspange
- 6.: Oberschenkelzugband
- 7.: Oberschenkelstützband
- 8.: Weitenverstellung
- 9.: Unterschenkelstützband
- 10.: Stützzunge
- 11.: Kniegelenkpolster
- 12.: Bewegungsgelenk
- 13.: Spangenverschluss
- 14.: Unterschenkelzugband
- 15.: Tibiapolster

## Patentansprüche

1. Knieorthese (1) zur Stabilisierung des Kniegelenks mit Rahmenteilen (2, 3) aus einem metallischen Werkstoff, die sich in einer Funktionsposition oberhalb und unterhalb des Kniegelenks jeweils längs eines Beinabschnittes erstrecken und mit zumindest jeweils einem Verschlussmittel (6, 7, 8, 9, 14) befestigt sind, das den Beinabschnitt formschlüssig lösbar umschlingt, wobei die einem Beinabschnitt zugeordneten Rahmenteile (2, 3) durch jeweils zumindest eine quer zum Beinabschnitt verlaufende feste Verbindungsspange (4, 5) verbunden sind und zwischen den ober- und unterhalb des Kniegelenks angeordneten Rahmenteilen (2, 3) einoder beidseitig des Kniegelenks jeweils schwenkbewegliche Verbindungsmittel (12) vorgesehen sind, deren Rotationsachsen im wesentlichen mit einer Hauptbewegungsachse des Kniegelenks übereinstimmen, **dadurch gekennzeichnet, dass** zumindest eine Verbindungsspange (4, 5) als separates Bauteil aus einem Werkstoff vorgesehen ist, der in einem vorübergehend plastifizierten Zustand unmittelbar an einen Beinquerschnitt des jeweiligen Beinabschnittes anpassbar ist.

2. Knieorthese (1) nach Anspruch 1, **dadurch gekennzeichnet, dass** die anpassbare Verbindungsspange (4, 5) als Tibiaspange dient.

3. Knieorthese (1) nach Anspruch 1, **dadurch gekennzeichnet, dass** der Werkstoff ein thermoplastischer Kunststoff mit einer Faserverstärkung, insbesondere mit einem Flächengebilde aus Kohlefaser ist.

4. Knieorthese nach Anspruch 1, **dadurch gekennzeichnet, dass** die Verbindungsspange lösbar mit den Rahmenteilen verbunden ist.
